# EUROPEAN PATENT SPECIFICATION

(11) **EP 3 689 568 B1**
(45) Date of publication and mention of the grant of the patent: **24.11.2021**
(21) Application number: 19214063.0
(22) Date of filing: 06.12.2019
(51) Int. Cl.: B28B 3/12, B28B 17/00, B30B 15/26, G01N 21/91, G01N 33/38, G01N 21/892

(54) **METHOD AND SYSTEM FOR MANUFACTURING CERAMIC TILES, WITH DETECTION OF INTEGRITY OF THE UNFIRED TILE**
VERFAHREN UND SYSTEM ZUR HERSTELLUNG VON KERAMIKFLIESEN MIT DETEKTION DER INTEGRITÄT DER UNGEBRANNTEN KACHEL
PROCÉDÉ ET SYSTÈME DE FABRICATION DE CARREAUX DE CÉRAMIQUE, AVEC DÉTECTION DE L'INTÉGRITÉ D'UN CARREAU NON SOUMIS À DES FLAMMES

(30) Priority: 04.02.2019 IT 201900001587
(43) Date of publication of application: 05.08.2020
(73) Proprietor: Granitifiandre Societa' Per Azioni, 42014 Castellarano (RE) (IT)
(72) Inventor: MARCHI, Domenico, 42031 BAISO (RE) (IT)
(74) Representative: Corradini, Corrado

(56) References cited:
- GB-A- 2 034 898
- IT-A1- MO20 120 106
- JP-A- H06 229 949
- JP-A- 2001 289 791

## Description

### Technical field

The present invention relates to a method and a system for manufacturing ceramic tiles, for example tiles and/or large ceramic tiles.

### Background art

It is known that manufacturing ceramic tiles generally requires providing a soft layer of ceramic powders, typically of atomized ceramic powders, which is suitable for successively being pressed by means of a suitable ceramic press in order to obtain an unfired tile of compacted ceramic powders.

The unfired tile thus obtained is then subjected to steps of drying, decorating and finally firing in a ceramic furnace so as to obtain a finished ceramic tile.

However, it may happen that the unfired tile outlet from the ceramic press at times has small defects, in particular small cracks or splits, especially at the side edges, which are not visible to the naked eye but which may cause the breaking of the ceramic tile during the successive firing step in the ceramic furnace.

In addition to the apparent consumption of material due to the reject of the ceramic tile, the occurrence of these cracks or splits only during the firing step also results in other drawbacks.

A first drawback consists of the fact that operators may intervene on the ceramic press only after a non-negligible number of probably faulty unfired tiles have already been made and are in the drying and decorating steps.

Another drawback consists of the fact that the breaking of a ceramic tile in the firing furnace may also be due to other causes, with the consequence that the operators at times are to perform lengthy and laborious investigations before understanding that the problem lies in the non-optimal operation of the ceramic press.

IT MO20 120 106 A1 discloses a method and a system for manufacturing ceramic tiles according to the preamble of claims 1 and 10.

### Description of the invention

It is therefore an object of the present invention to resolve the mentioned drawbacks of the known technique within the scope of a simple, rational and relatively affordable solution.

This and other objects are reached thanks to the characteristics of the invention as set forth in the independent claims. The dependent claims outline preferred and/or particularly advantageous aspects of the invention.

In particular, one embodiment of the present invention makes available a method for manufacturing ceramic tiles, comprising the steps of:
- depositing a soft layer of ceramic powder on a support element,
- pressing said soft layer against said support element, thus creating an unfired tile of compacted ceramic powder, and
- firing the unfired tile inside a ceramic furnace,
wherein said method also comprises a process of monitoring the integrity of the unfired tile, which comprises the steps of:
- applying, on at least one portion of the unfired tile, a contrast liquid suitable for modifying the colour of said portion of the unfired tile,
- acquiring at least one image of said portion of the unfired tile after the application of the contrast liquid and before the firing step (preferably before the possible drying and/or decorating steps), and
- detecting if said image comprises at least one line of contrasting colour with respect to the colour taken on by said portion of the unfired tile following the application of the contrast liquid.

Thanks to this solution, the method advantageously is capable of recognizing if, after the pressing step, the portion treated with the contrast liquid has a defect, for example a crack or a split, which could cause the breaking or at least significant damage in the unfired tile during the successive firing step.

Indeed, the colour taken on by the portion treated with the contrast liquid would substantially be uniform should there be no defect. Vice versa, should the portion treated with the contrast liquid have a crack or split, the absorption of the contrast liquid at said crack or split would be different with respect to the absorption of the same contrast liquid in the surrounding areas, thus creating a line of contrasting colour, for example lighter or darker, with respect to the colour taken on by said surrounding areas.

Therefore, if the presence of such a line of contrasting colour is detected in the image, it is effectively possible to identify, within the portion treated with the contrast liquid, that the unfired tile of compacted powder has a defect, typically a crack or a split.

If no line of contrasting colour is detected, the unfired tile may be subjected to the firing step in the ceramic furnace.

If instead a line of contrasting colour is detected, the method may provide various actions.

One of these actions may simply be generating an alarm suitable for warning that a defect was identified in the unfired tile, for example to allow the operators in charge of production to promptly intervene.

This alarm may be for example, a warning light, an acoustic signal or a message that may be sent by email or by means of any other communication system.

Another action which may be implemented in response to the detection of the line of contrasting colour is the one of discarding the unfired tile on which said line was detected, thus avoiding it from being subjected to the firing step and possibly also from the drying and decorating steps.

Other actions may relate to adjusting and/or controlling the step of depositing the soft layer and/or the pressing step, for example by modifying one or more operating parameters of the devices intended to carry out said steps.

Each of these possible actions may be manually carried out by an operator (or in any case with the intervention thereby) or automatically by a unit for electronically controlling the manufacturing process.

According to a preferred aspect of the invention, the contrast liquid may be an organic liquid.

Thanks to this solution, during the successive firing step in the ceramic furnace, the contrast liquid evaporates and/or burns, substantially without leaving any trace on the finished ceramic tile.

In general, the contrast liquid may be any liquid capable of wetting the portion of the unfired tile on which it is applied so as to modify the colour thereof with respect to the colour that the unfired tile had before such application.

It is therefore not excluded for the contrast liquid to itself have a neutral or transparent colour, as water.

However, it is preferable for the contrast liquid to be a colourant in the liquid state having a characteristic colour, for example but not exclusively blue, red or green.

In this manner, it is indeed possible to cause the line indicating a possible defect, typically a crack or split, to stand out more.

Another aspect of the invention provides for the portion of the unfired tile on which the contrast liquid is applied to be localized at a side edge of the unfired tile itself.

The side edges of the unfired tile indeed are the areas most subject to the occurrence of cracks or splits, hence it may be sufficient to investigate only these critical areas to in any case have a reasonable certainty that the unfired tile does not have any defects.

According to another aspect of the invention, the line of contrasting colour to be detected in the image may be a substantially continuous and open line, for example substantially shaped as a broken line, which extends transversely, for example in substantially orthogonal direction, with respect to the edge of the unfired tile, for example from the edge of the unfired tile towards the centre thereof.

Thanks to this solution, it advantageously is possible to increase the reliability of the method by detecting in the image only the possible presence of lines which actually have the shape of a crack or a split, and therefore avoiding lines of contrasting colour generated by other phenomena - for example by the application methods of the contrast liquid - from being erroneously identified as defects in the lines of contrasting colour.

A further aspect of the invention provides for the contrast liquid to be applied on a plurality of different portions of the unfired tile, at least one image to be acquired of each of said portions of the unfired tile after the application of the contrast liquid and before the firing step, and detecting if at least one of said images comprises said line of contrasting colour.

By carrying out the steps of applying the contrast liquid, acquiring the image and detecting the line on a plurality of different portions of the unfired tile, it advantageously is possible to assess the integrity of the tile in a more extended and complete manner.

For example, these steps may be carried out on at least two portions of the unfired tile which are localized at two opposed edges of the unfired tile itself.

According to an embodiment of the invention, the support element for the soft layer of ceramic powder may be a slidable conveyor belt.

Thanks to this solution, it advantageously is possible to make large ceramic tiles by means of relatively compact and economically acceptable machines and equipment. In this context, the step of pressing the soft layer may occur while the conveyor belt is sliding.

In this manner, a continuous pressing is carried out which allows making a continuous unfired tile, which may then be divided, by means of convenient cutting members, into individual unfired tiles having defined sizes, which may successively be subjected to the usual drying and decorating steps, before passing to the firing step inside the ceramic furnace.

In this case, the steps of applying the contrast liquid and acquiring the image may be carried out directly on the continuous unfired tile, before the division into individual tiles.

However, it is not excluded in other embodiments for the step of pressing the soft layer to occur while the conveyor belt is stopped, within the realm of a discontinuous pressing.

This type of pressing may provide for example, pressing a sequence of individual soft layers of ceramic powders advancing by steps on a slidable conveyor belt so as to obtain, at the outlet of the already separate unfired tiles of compacted ceramic powder, after a possible step of trimming the edges, that they may be dried, decorated and finally fired in the ceramic furnace.

Systems that operate in this manner are for example, the ones commercially known as GEA System or Supera Siti B&T.

In this case, the steps of applying the contrast liquid and acquiring the image may be carried out on each unfired tile outlet from the pressing step.

In both cases outlined above, the steps of applying the contrast liquid and of acquiring the image may both occur while the unfired tile is caused to advance according to a preset advancement direction, preferably parallel to or coinciding with the sliding direction of the conveyor belt.

In particular, the application of the contrast liquid may occur by means of a fixed applicator, for example a dispensing nozzle, that applies, for example by spraying, the contrast liquid on the unfired tile progressively advancing, thus creating thereon a band of different colour that extends parallel to the advancement direction.

The acquisition of the image may occur by means of a fixed camera or video camera, which is suitable for framing in sequence, successive portions of said different-coloured band as the unfired tile continues advancing.

Thanks to this solution, the identification of possible defects does not introduce dead times and therefore does not slow down the production process.

The present invention also makes available a system for making ceramic tiles, comprising:
- a device for depositing a soft layer of ceramic powder on a support element,
- a ceramic press for pressing said soft layer against said support element, thus creating an unfired tile of compacted ceramic powder, and
- a ceramic furnace suitable for firing the unfired tile,
the system comprises an apparatus for monitoring the integrity of the unfired tile, which comprises:
- a device for applying, on at least one portion of the unfired tile, a contrast liquid suitable for modifying the colour of said portion of the unfired tile,
- an optical device suitable for acquiring at least one image of said portion of the unfired tile after the application of the contrast liquid and before the firing step (preferably before the possible drying and/or decorating steps), and
- an electronic processing unit connected with said optical device and configured to detect if said image comprises at least one line of contrasting colour with respect to the colour taken on by said portion (P) of the unfired tile following the application of the contrast liquid.

This system substantially achieves the same effects as the method outlined above, in particular the one of identifying the presence of possible defects, such as for example cracks or splits, in the unfired tile before this is subjected to the firing step.

Naturally, all the accessory aspects of the invention that were described above in relation to the method are intended also applicable to the relative system.

### Brief description of the drawings

Further features and advantages of the invention will be more apparent after reading the following description provided by way of a non-limiting example, with the aid of the accompanying drawings.

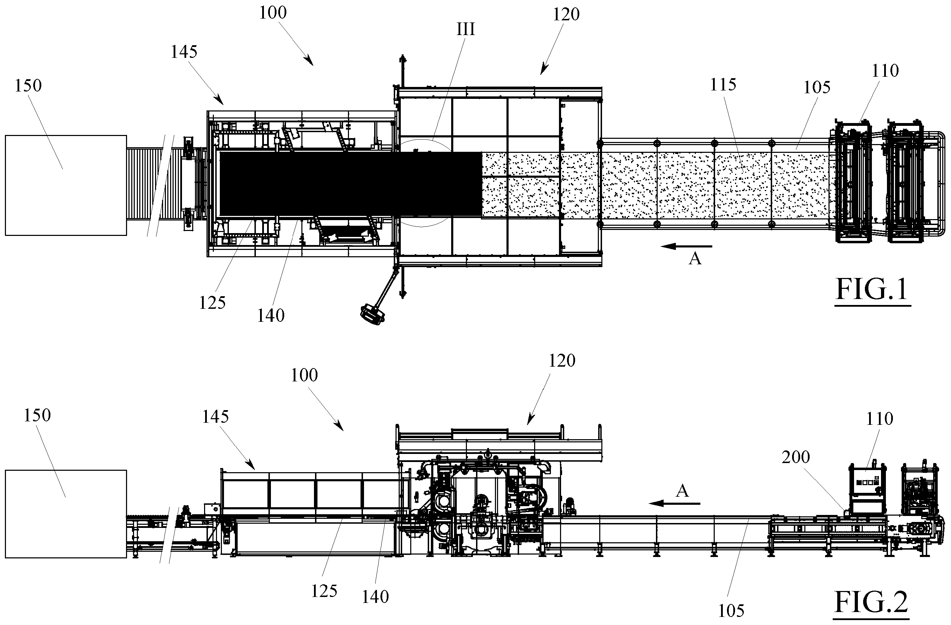

Figure 1 is a schematic plan and top view of a system suitable for manufacturing tiles and/or ceramic tiles according to an embodiment of the present invention.
Figure 2 is a side view of the system of figure 1.
Figure 3 is detail III-III indicated in figure 1, shown on enlarged scale.
Figure 4 is a side view of the detail of figure 3.
Figures 5 and 6 show two possible images of the unfired tile acquired during the execution of a method according to the present invention.

### Detailed description

The mentioned drawings show a system 100 for making ceramic tiles, typically large ceramic tiles.

The ceramic tiles obtained with the system 100 may be intended to cover floors, walls or any other architectural and non-architectural surface.

The system 100 comprises a support element, in the example a conveyor belt 105 suitable for sliding in a preset advancement direction A, preferably horizontal and rectilinear.

The conveyor belt 105 may slide along the advancement direction A at a preset speed, for example but not necessarily at a speed comprised between 3 m/min (metres per minute) and 5 m/min (end values included).

At least one dispensing device 110 is placed above the conveyor belt 105, for example a hopper or any other device suitable for the purpose, which is suitable for pouring a ceramic powder onto the conveyor belt 105.

The ceramic powder may be obtained by grinding a mixture of raw ceramic materials, among which for example, clays, talc, silica, feldspathic minerals, carbonates, micas, vitreous materials, etc.

The mixture of raw ceramic materials may be dry ground, thus directly obtaining the ceramic powder, or it may be wet ground and then be subjected to a drying process inside an atomizer.

The ceramic powder in the present disclosure preferably is obtained with the second method outlined above and therefore takes the name "atomized".

The ceramic powder preferably is "neutral", i.e. is obtained without the addition of pigments or other colourants so that the colour thereof (generally light grey) is the natural one of the mixture of raw ceramic materials that were ground and atomized. The dispensing device 110 is controlled to dispense the ceramic powder over the conveyor belt 105 while the latter is sliding so as to create thereon a soft layer 115 of ceramic powder having a given thickness and that progressively advances in the advancement direction A.

Preferably, the dispensing of the ceramic powder by the dispensing device 110 occurs in continuous manner during the sliding of the conveyor belt 105 so that the soft layer 115 correspondingly is a single continuous layer of ceramic powder without interruption.

However, it is not excluded in other embodiments for the dispensing of the ceramic powder by the dispensing device 110 to occur in intermittent manner during the sliding of the conveyor belt 105, thereby creating a sequence of soft layers 115 having defined length and separate from one another.

It is worth noting that "soft" layer in ceramic jargon generally means a non-pressed layer of ceramic powder.

The width of the soft layer 115 of ceramic powder, i.e. the dimension thereof in direction orthogonal to the advancement direction A and to the thickness thereof, may be less than or equal to the width of the conveyor belt 105 and clearly also depends on the transverse extension of the dispensing device 110.

The system 100 may comprise, downstream of the dispensing device 110 with respect to the advancement direction A, one or more levelling devices 200 which are placed above the conveyor belt 105 so as to adjust and make uniform the thickness of the soft layer 115.

These levelling devices 200, which may be of suction type, preferably are associated with specific actuator members (not illustrated), which are suitable for modifying the distance thereof with respect to the conveyor belt 105.

By advancing on the conveyor belt 105, the soft layer 115 of ceramic powder is transferred to a ceramic press 120, which is suitable for pressing the soft layer 115 so as to obtain an unfired tile 125 of compacted ceramic powder.

The ceramic press 120 may be a continuous press suitable for pressing the soft layer 115 directly on the conveyor belt 105 and during the advancement thereof.

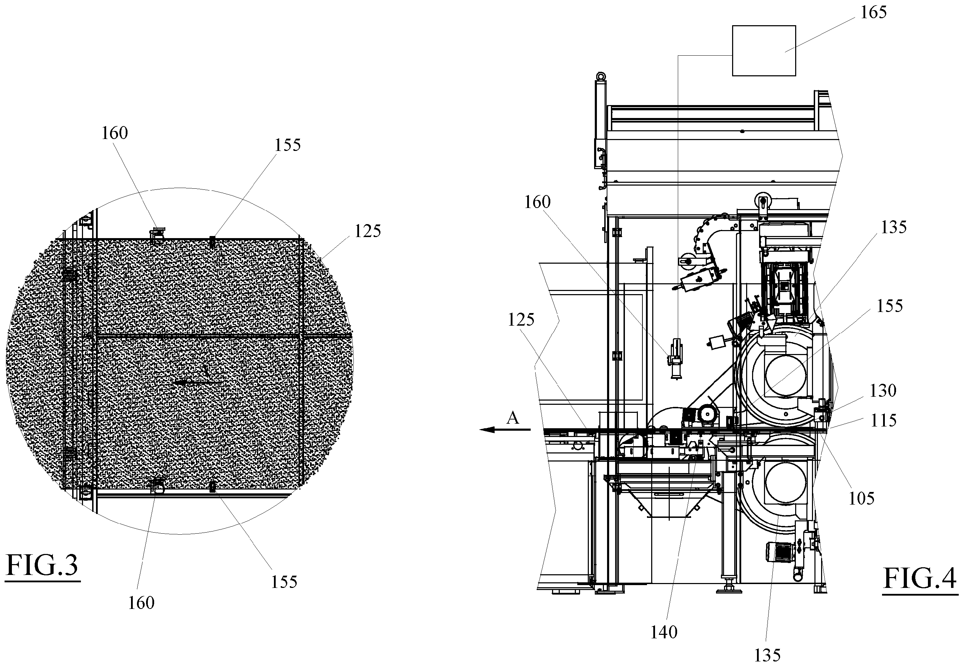

In practice, the ceramic press 120 may comprise a slidable tamping belt 130 (see fig. 4), which overlaps the soft layer 115 of ceramic powder and advances in the same direction and at the same speed as the conveyor belt 105.

The conveyor belt 105 and the tamping belt 130 are caused to pass through two tamping rollers 135, or through two arrangements of tamping rollers, which push the tamping belt 130 towards the conveyor belt 105 and thus allow them to press the soft layer 115, thus obtaining an outgoing unfired tile 125 of compacted ceramic powders. The unfired tile 125 may leave the conveyor belt 105 immediately downstream of the tamping rollers 135 and progressively move onto another belt, for example onto a roller conveyor 140, which may be arranged coplanar to the conveyor belt 105 and be suitable for causing the unfired tile 125 to advance in the same advancement direction A, at least up to the complete outlet thereof from the ceramic press 120.

The pressure with which the soft layer 115 is pressed may vary according to the materials used, from a minimum of 150 kg/cm² (kilograms per square centimetre) to a maximum of 600 kg/cm².

For example, in the case of porcelain stoneware, the pressure exerted by the ceramic press 120 on the soft layer 115 of ceramic powder may be comprised between 300 kg/cm² and 450 kg/cm², more preferably between 350 kg/cm² and 450 kg/cm² (end values included).

If the dispensing device 110 dispenses the ceramic powder in intermittent manner, thus creating a sequence of separate soft layers 115 on the conveyor belt 105, a sequence of already separate individual unfired tiles 125 correspondingly is obtained outlet from the ceramic press 120.

In the preferred case instead in which the dispensing of the ceramic powder by the dispensing device 110 is continuous and the soft layer 115 correspondingly is a continuous layer without interruptions, the unfired tile 125 outlet from the ceramic press 120 is also a continuous tile without interruptions.

In this case, the system 100 may further comprise, downstream of the ceramic press 120, a cutting device 145 suitable for dividing the continuous unfired tile 125 into individual tiles having defined sizes.

In any case, the operation of the ceramic press 120 outlined above preferably always occurs in continuous manner, i.e. with the conveyor belt 105 and the tamping belt 130 continuously sliding.

However in other embodiments, the ceramic press 120 could be a discontinuous press, for example of the type commercially known as GEA System or Supera Siti B&T.

In this case, the conveyor belt 105 may be caused to advance by modest steps, having each advancement step followed by a stopping step.

The dispensing device 110 may be controlled to dispense the ceramic powder over the conveyor belt 105 only during the advancement steps by interrupting the dispensing during the stopping step so as to obtain a sequence of individual soft layers 115 having preset length and separate from one another by a given distance.

At the same time, one soft layer 115 at a time of this sequence would be stopped at the ceramic press 120 during each stopping step of the conveyor belt 105. Therefore, the ceramic press 120 could comprise pressing means which, while the soft layer 115 of ceramic powder is stopped, are suitable for directly pressing it on the conveyor belt 105.

These pressing means may comprise for example, an abutment plate placed above the soft layer 115 of ceramic powder, and suitable hydraulic systems which are suitable for raising the portion of conveyor belt 105 on which there is the soft layer 115, pressing the latter against the abutment plate.

Then, the conveyor belt 105 is lowered and caused to advance to move away the unfired tile 125 thus obtained and bring a new soft layer 115 of ceramic powder at the ceramic press 120.

Regardless of the type of ceramic press 120 used, the unfired tiles 125 obtained may then be sent to other operating stations (not shown) where they are subjected to further steps of the production process, among which for example a drying step, a decorating step and/or a possible glazing step.

Finally, the unfired tiles 125 are transported into a ceramic furnace 150 where they are subjected to a firing step, for example at a maximum temperature which may reach values comprised between 1000°C and 1300°C.

The system 100 also comprises a monitoring apparatus that generally is configured to verify if the unfired tile 125, i.e. the tile not yet subjected to the firing step, coming from the ceramic press 120 is intact or has defects, for example cracks or splits that could cause the breaking thereof in the ceramic furnace 150.

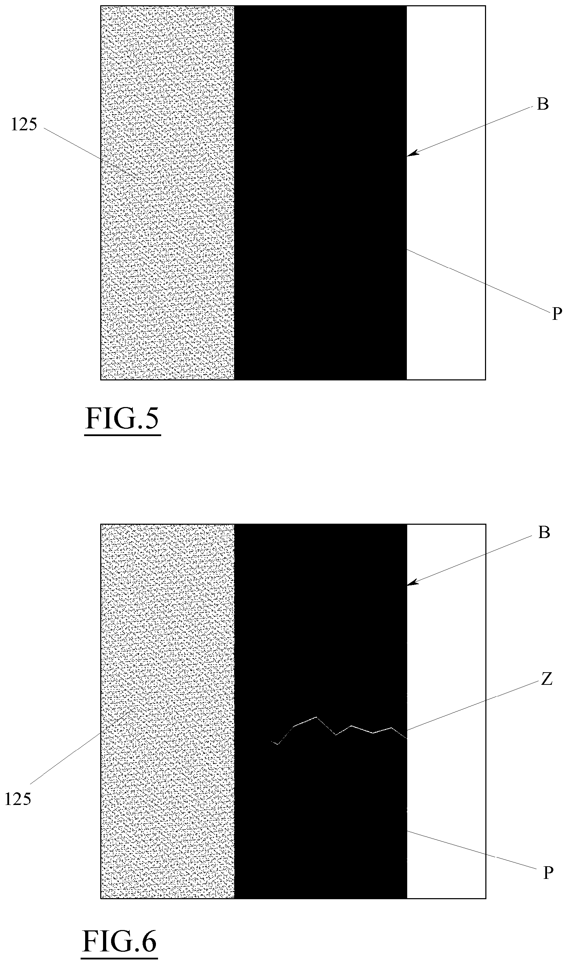

In general, such a verification firstly provides applying, on at least one portion P of the unfired tile 125, a contrast liquid suitable for modifying the colour of said portion P of the unfired tile 125 (see figures 5 and 6).

The unfired tile 125 outlet from the ceramic press 120 indeed normally has the colour of the starting ceramic powder which as mentioned above, may be of the "neutral" type and therefore have a substantially light grey colour.

The contrast liquid may be a colourant in the liquid state having a characteristic colour thereof that is different from the colour of the unfired tile 125, for example but not exclusively blue, red or green.

In general, the contrast liquid may however be any liquid capable of wetting the portion of the unfired tile 125 on which it is applied so as to modify the colour thereof with respect to the colour that the unfired tile 125 had before such application.

The adjective "coloured" used in the present disclosure is therefore to be intended as "of different colour" and not necessarily as "obtained by means of the addition of a colourant or pigment".

In this sense, it is therefore not strictly necessary for the contrast liquid to have its own characteristic colour, it possibly also being a clear liquid like water.

In any case, it always is preferable for the contrast liquid to be an organic liquid so that it may completely or almost completely evaporate and/or burn without leaving substantial traces on the finished ceramic tile during the successive firing step in the ceramic furnace 150.

A particularly effective organic contrast liquid is by way of non-limiting example, methylene blue.

The contrast liquid may be applied on one of the flat faces of the unfired tile 125 alone, for example on the upper face of the unfired tile 125, i.e. on the face opposite to the one that is resting on the conveyor belts downstream of the ceramic press 120. Theoretically, the contrast liquid could be applied over the whole surface of this face. However, to limit the consumption of the contrast liquid, it preferably is applied only at one or more limited areas of the aforesaid surface, i.e. in the ones in which the probability of detecting defects generally is higher.

In particular, these areas normally are localized at side edges of the unfired tile 125, for example at and along the two opposed longitudinal edges that extend parallel to the advancement direction A of the ceramic powder in the ceramic press 120.

From a practical viewpoint, the monitoring apparatus may comprise for example, two applicators 155, which may be installed in fixed position downstream of the ceramic press 120 with respect to the advancement direction A, preferably between the ceramic press 120 and the possible cutting device 145 (see figures 3 and 4).

Each applicator 155 is suitable for applying the contrast liquid over the upper face of the unfired tile 125 at a respective longitudinal edge as said unfired tile 125 advances on the conveyor belts in the advancement direction A.

In particular, each applicator 155 may be a dispensing nozzle suitable for spraying the contrast liquid over the unfired tile 125.

The application of the contrast liquid by each applicator 155 may occur in continuous manner, thus creating, on the upper face of the unfired tile 125, a coloured band that extends parallel to the advancement direction A and without interruptions, for the whole length of the unfired tile 125 itself.

Alternatively, the application of the contrast liquid by each applicator 155 could occur in intermittent manner so as to create, on the upper face of the unfired tile 125, a plurality of coloured traces that are individually separate from one another.

Naturally, should the ceramic press 120 generate a sequence of already separate unfired tiles 125, the application of the contrast liquid, be it continuous or intermittent, could be suspended when an empty space passes between two successive unfired tiles 125 at each applicator 155.

After the application of the contrast liquid, the verification provides acquiring at least one image of each portion P of the unfired tile 125 on which the contrast liquid was applied.

The acquisition of this image may occur by means of at least one optical device 160, such as for example a camera or a video camera, which is suitable for framing and acquiring images of the portion P of the unfired tile 125 previously treated with the contrast liquid.

With reference to the example shown in figures 3 and 4, the system may for example, comprise two optical devices 160, which may be installed in fixed position downstream of the ceramic press 120, preferably between the applicators 155 of the contrast liquid and the possible cutting device 145, with respect to the advancement direction A of the unfired tile 125.

Each of these optical devices 160 may be oriented so as to frame a portion of space which, as the unfired tile 125 advances in the advancement direction A, is crossed by successive portions of the coloured band (or by the coloured traces) that was obtained on the unfired tile 125 by a respective applicator 155.

Although there are two applicators 155 and two optical devices 160 in this example, other embodiments of the system 100 could comprise one applicator 155 alone and/or one optical device 160 alone, or a greater number of applicators 155 and/or of optical devices 160.

In any case, each image acquired by each optical device 160 may contain a two-dimensional graphic reproduction of at least a part of the unfired tile 125 which includes at least one stretch of the coloured band (or of the coloured trace) made by the respective applicator 155, as diagrammatically shown in figures 5 and 6.

Each optical device 160 may possibly be configured, for each unfired tile 125, to acquire, while the unfired tile 125 continues advancing, a plurality of images at successive time intervals and with a determined frequency so as to obtain a plurality of images which overall include the graphic reproduction of the whole coloured band (or of all the coloured traces) made by the respective applicator 155.

The verification at this point provides verifying, for each of the images acquired, if said image comprises at least one line having a contrasting colour with respect to the colour that was given to portion P of the unfired tile 125 that was treated with the contrast liquid.

Indeed, should the coloured portion P of the unfired tile 125 not have any defects, the colour given to it by the application of the contrast liquid would substantially be uniform, as diagrammatically shown in figure 5.

Vice versa, should the coloured portion P of the unfired tile 125 have a defect, e.g. a crack or split, the absorption of the contrast liquid at said crack or split would be different with respect to the absorption of the same contrast liquid in the surrounding areas of the coloured portion, thus creating a line Z of contrasting colour, for example lighter or darker, with respect to the colour taken on by said surrounding areas, as diagrammatically shown in figure 6.

The presence or absence of this line of contrasting colour Z naturally is also reproduced in the image required.

Therefore, if the line of contrasting colour Z is detected in the image, it means that a defect, typically a crack or a split, is in the real unfired tile 125.

The line of contrasting colour Z to be detected in the image acquired may also require meeting one or more further requirements to increase the reliability of the verification and reduce the risk of false identifications.

A first of these requirements may be that line Z be inside the perimeter of the area of the image that represents the coloured portion P of the unfired tile 125 so as to exclude for example, the transition lines with the remaining untreated parts of the unfired tile 125 or with possible elements placed in the background of the images.

A second of these requirements may be that line Z be a substantially continuous and open line, for example substantially shaped as a broken line.

Another of these requirements may be that line Z extend transversely, for example in substantially orthogonal direction with respect to the depiction in the drawing of edge B of the unfired tile 125 at which the contrast liquid was applied, for example a line that extends from the depiction of edge B of the unfired tile 125 towards the centre of the unfired tile itself.

Thanks to one or more of these further requirements, it advantageously is possible to detect in the image only the possible presence of lines Z that actually have the shape of a crack or a split, and therefore avoiding lines of contrasting colour generated by other phenomena, for example by the imperfect of not completely uniform application of the contrast liquid, from being erroneously identified as defects in the lines of contrasting colour.

The analysis of each image, which aims to detect a possible line of contrasting colour Z, may be carried out by an electronic control unit 165 which may be connected with each optical device 160 so as to receive the images acquired, and may be configured to execute a recognition software of the lines of contrasting colour Z in the images received.

If no line of contrasting colour Z is detected in the image(s), the unfired tile 125 may be caused to normally proceed towards the ceramic furnace 150, possibly after passing through a drying station and/or a decorating and/or glazing station.

If instead a line of contrasting colour Z is detected, it is identified that the unfired tile 125 has a defect and the method may possibly provide carrying out various actions. One of these actions may simply be generating an alarm suitable for warning that a defect was identified in the unfired tile 125, for example to allow the operators in charge of the system 100 to promptly intervene.

This alarm may be for example, a warning light (for example, a light turning on), an acoustic signal (for example, the activation of an alarm) or a message that may be sent by email or by means of any other communication system.

Another possible action which may be implemented in response to the detection of the line of contrasting colour Z is the one of discarding the unfired tile 125 on which said line was detected.

Other actions may relate to adjusting one or more operating parameters of the depositing step of the soft layer 115 and/or the pressing step, among which for example one or more operating parameters of the dispensing device 110, the distance of the levelling devices 200 with respect to the conveyor belt 105 and one or more operating parameters of the ceramic press 120, such as for example the pressure exerted by the ceramic press 120 on the soft layer 115 of ceramic powder.

Each of these possible actions may be manually carried out by an operator (or in any case with the intervention by the operator) or automatically by an electronic unit of the system 100 operatively connected with the aforesaid devices, for example by the electronic unit 165 or by another electronic unit connected therewith.

In general, all the process steps described above may be automatically managed by a computer configured to control all the various components of the system 100 according to the methods described.

It is also worth noting that each of the conveyor belts mentioned above is to be intended as the operational stretch of said conveyor belt. Each conveyor belt indeed generally is wound about a plurality of rollers or idler wheels (at least one of which being motorized) having horizontal axis, which engage it to slide along a closed path that comprises an operational stretch, in which the conveyor belt is facing upwards to support the ceramic powder, and a return stretch in which the conveyor belt is facing downwards. For brevity of disclosure, each conveyor belt in the preceding description was identified with an operational stretch thereof and accordingly, the advancement direction and the speed indicated refer to the sliding direction and the speed of the conveyor belt in said operational stretch.

Obviously, an expert in the field may make several technical-applicative modifications to the system 100 and to the relative operating method, without departing from the scope of the invention as hereinbelow claimed.

## Claims

1. A method for manufacturing ceramic tiles, comprising the steps of:
- depositing a soft layer (115) of ceramic powder on a support element (105),
- pressing said soft layer (115) against said support element (105), thus creating an unfired tile (125) of compacted ceramic powder, and
- firing the unfired tile (125) inside a ceramic furnace (150),
**characterized in that** it comprises a process of monitoring the integrity of the unfired tile, which includes the steps of
- applying, on at least one portion (P) of the unfired tile (125), a contrast liquid suitable for modifying the colour of said portion (P) of the unfired tile (125),
- acquiring at least one image of said portion (P) of the unfired tile (125) after the application of the contrast liquid and before the firing step, and
- detecting if said image comprises at least one line (Z) of contrasting colour with respect to the colour taken on by said portion (P) of the unfired tile (125) following the application of the contrast liquid.

2. A method according to claim 1, wherein the contrast liquid is an organic liquid.

3. A method according to claim 1 or 2, wherein the contrast liquid is a colourant in the liquid state.

4. A method according to any one of the preceding claims, wherein the portion (P) of the unfired tile (125) on which the contrast liquid is applied is localized at a side edge of the unfired tile (125).

5. A method according to claim 4, wherein the line (Z) of contrasting colour is a substantially continuous and open line, which extends transversely with respect to the edge of the unfired tile (125).

6. A method according to any one of the preceding claims, wherein the contrast liquid is applied on a plurality of different portions (P) of the unfired tile (125), in which at least one image is acquired of each of said portions (P) of the unfired tile (125) after the application of the contrast liquid and before the firing step, and wherein it is detected if at least one of said images comprises said line (Z) of contrasting colour.

7. A method according to any one of the preceding claims, wherein the support element for the soft layer (115) of ceramic powder is a slidable conveyor belt (105).

8. A method according to claim 7, wherein the step of pressing the soft layer (115) occurs while the conveyor belt (105) is sliding.

9. A method according to claim 7 or 8, wherein the steps of applying the contrast liquid and of acquiring the image both occur while the unfired tile (125) is caused to advance according to a preset advancement direction (A).

10. A system (100) for making ceramic tiles, comprising:
- a device (110) for depositing a soft layer (115) of ceramic powder on a support element (105),
- a ceramic press (120) for pressing said soft layer against said support element (105), thus creating an unfired tile (125) of compacted ceramic powder, and
- a ceramic furnace (150) suitable for firing the unfired tile (125),
**characterized in that** it comprises an apparatus for monitoring the integrity of the unfired tile, which comprises:
- a device (155) for applying, on at least one portion (P) of the unfired tile (125), a contrast liquid suitable for modifying the colour of said portion of the unfired tile (125),
- an optical device (160) suitable for acquiring at least one image of said portion (P) of the unfired tile (125) after the application of the contrast liquid and before the firing step, and
- an electronic processing unit (165) connected with said optical device (160) and configured to detect if said image comprises at least one line (Z) of contrasting colour with respect to the colour taken on by said portion (P) of the unfired tile (125) following the application of the contrast liquid.

## Patentansprüche

1. Verfahren zur Herstellung von Keramikfliesen, das die folgenden Schritte umfasst:
- Aufbringen einer weichen Schicht (115) aus Keramikpulver auf ein Trägerelement (105),
- Pressen der weichen Schicht (115) gegen das Trägerelement (105), wodurch eine ungebrannte Fliese (125) aus verdichtetem Keramikpulver entsteht, und
- Brennen der ungebrannten Fliese (125) in einem Keramikofen (150),
**dadurch gekennzeichnet, dass** es ein Verfahren zur Überwachung der Unversehrtheit der ungebrannten Fliese umfasst, das die folgenden Schritte umfasst
- Auftragen einer Kontrastflüssigkeit auf mindestens einen Abschnitt (P) der ungebrannten Fliese (125), die geeignet ist, die Farbe des genannten Abschnitts (P) der ungebrannten Fliese (125) zu verändern,
- Erfassen mindestens eines Bildes des Abschnitts (P) der ungebrannten Fliese (125) nach dem Auftragen der Kontrastflüssigkeit und vor dem Brennschritt, und
- Feststellen, ob das Bild mindestens eine Linie (Z) von kontrastierender Farbe in Bezug auf die Farbe aufweist, die der Abschnitt (P) der ungebrannten Fliese (125) nach dem Auftragen der Kontrastflüssigkeit angenommen hat.

2. Verfahren nach Anspruch 1, wobei die Kontrastflüssigkeit eine organische Flüssigkeit ist.

3. Verfahren nach Anspruch 1 oder 2, wobei die Kontrastflüssigkeit ein Farbstoff in flüssigem Zustand ist.

4. Verfahren nach einem der vorhergehenden Ansprüche, wobei der Abschnitt (P) der ungebrannten Fliese (125), auf den die Kontrastflüssigkeit aufgetragen wird, an einer Seitenkante der ungebrannten Fliese (125) lokalisiert ist.

5. Verfahren nach Anspruch 4, wobei die Linie (Z) der Kontrastfarbe eine im Wesentlichen durchgehende und offene Linie ist, die sich quer zum Rand der ungebrannten Fliese (125) erstreckt.

6. Verfahren nach einem der vorhergehenden Ansprüche, wobei die Kontrastflüssigkeit auf eine Vielzahl verschiedener Abschnitte (P) der ungebrannten Fliese (125) aufgetragen wird, wobei von jedem der Abschnitte (P) der ungebrannten Fliese (125) nach dem Auftragen der Kontrastflüssigkeit und vor dem Brennschritt mindestens ein Bild aufgenommen wird, und wobei detektiert wird, ob mindestens eines der Bilder die Linie (Z) mit kontrastierender Farbe enthält.

7. Verfahren nach einem der vorhergehenden Ansprüche, wobei das Trägerelement für die weiche Schicht (115) aus Keramikpulver ein verschiebbares Förderband (105) ist.

8. Verfahren nach Anspruch 7, wobei der Schritt des Pressens der weichen Schicht (115) bei gleitendem Förderband (105) erfolgt.

9. Verfahren nach Anspruch 7 oder 8, wobei die Schritte des Auftragens der Kontrastflüssigkeit und des Erfassens des Bildes beide erfolgen, während die ungebrannte Fliese (125) veranlasst wird, sich gemäß einer voreingestellten Vorschubrichtung (A) vorwärts zu bewegen.

10. System (100) zur Herstellung keramischer Fliesen, umfassend:
- eine Vorrichtung (110) zum Aufbringen einer weichen Schicht (115) aus Keramikpulver auf ein Trägerelement (105),
- eine Keramikpresse (120) zum Pressen der weichen Schicht gegen das Trägerelement (105), wodurch eine ungebrannte Fliese (125) aus verdichtetem Keramikpulver entsteht, und
- einen Keramikofen (150), der zum Brennen der ungebrannten Fliese (125) geeignet ist,
**dadurch gekennzeichnet, dass** es eine Vorrichtung zur Überwachung der Unversehrtheit der ungebrannten Ziegel umfasst, die Folgendes umfasst:
- eine Vorrichtung (155) zum Auftragen einer Kontrastflüssigkeit auf mindestens einen Abschnitt (P) der ungebrannten Fliese (125), die geeignet ist, die Farbe des genannten Abschnitts der ungebrannten Fliese (125) zu verändern,
- eine optische Vorrichtung (160), die geeignet ist, mindestens ein Bild des Abschnitts (P) der ungebrannten Fliese (125) nach dem Auftragen der Kontrastflüssigkeit und vor dem Brennschritt zu erfassen, und
- eine elektronische Verarbeitungseinheit (165), die mit der optischen Vorrichtung (160) verbunden und so konfiguriert ist, dass sie erkennt, ob das Bild mindestens eine Linie (Z) mit einer kontrastierenden Farbe in Bezug auf die Farbe aufweist, die der Abschnitt (P) der ungebrannten Fliese (125) nach dem Auftragen der Kontrastflüssigkeit annimmt.

## Revendications

1. Procédé pour la fabrication de carreaux de céramique, comprenant les étapes consistant à :
- déposer une couche tendre (115) de poudre céramique sur un élément de support (105),
- presser ladite couche tendre (115) contre ledit élément de support (105), en créant ainsi un carreau non cuit (125) de poudre céramique compactée, et
- cuire le carreau non cuit (125) à l'intérieur d'un four à céramique (150), **caractérisé en ce qu'**il comprend un processus de surveillance de l'intégrité du carreau non cuit, qui inclut les étapes consistant à
- appliquer, sur au moins une partie (P) du carreau non cuit (125), un liquide de contraste approprié pour modifier la couleur de ladite partie (P) du carreau non cuit (125),
- acquérir au moins une image de ladite partie (P) du carreau non cuit (125) après l'application du liquide de contraste et avant l'étape de cuisson, et
- détecter si ladite image comprend au moins une ligne (Z) de couleur contrastée par rapport à la couleur adoptée par ladite partie (P) du carreau non cuit (125) à la suite de l'application du liquide de contraste.

2. Procédé selon la revendication 1, dans lequel le liquide de contraste est un liquide organique.

3. Procédé selon la revendication 1 ou 2, dans lequel le liquide de contraste est un colorant à l'état liquide.

4. Procédé selon l'une quelconque des revendications précédentes, dans lequel la partie (P) du carreau non cuit (125) sur laquelle le liquide de contraste est appliqué est localisée au niveau d'un bord latéral du carreau non cuit (125).

5. Procédé selon la revendication 4, dans lequel la ligne (Z) de couleur contrastée est une ligne sensiblement continue et ouverte, qui s'étend transversalement par rapport au bord du carreau non cuit (125).

6. Procédé selon l'une quelconque des revendications précédentes, dans lequel le liquide de contraste est appliqué sur une pluralité de différentes parties (P) du carreau non cuit (125), dans lequel au moins une image est acquise de chacune desdites parties (P) du carreau non cuit (125) après l'application du liquide de contraste et avant l'étape de cuisson, et dans lequel il est détecté si au moins l'une desdites images comprend ladite ligne (Z) de couleur contrastée.

7. Procédé selon l'une quelconque des revendications précédentes, dans lequel l'élément de support pour la couche tendre (115) de poudre céramique est une bande transporteuse (105) coulissante.

8. Procédé selon la revendication 7, dans lequel l'étape de pressage de la couche tendre (115) a lieu tandis que la bande transporteuse (105) coulisse.

9. Procédé selon la revendication 7 ou 8, dans lequel les étapes d'application du liquide de contraste et d'acquisition de l'image ont lieu toutes les deux tandis que le carreau non cuit (125) est amené à avancer selon une direction d'avancement prédéfinie (A).

10. Système (100) pour la fabrication de carreaux de céramique, comprenant :
- un dispositif (110) pour déposer une couche tendre (115) de poudre céramique sur un élément de support (105),
- une presse à céramique (120) pour presser ladite couche tendre contre ledit élément de support (105), en créant ainsi un carreau non cuit (125) de poudre céramique compactée, et
- un four à céramique (150) approprié pour cuire le carreau non cuit (125), **caractérisé en ce qu'**il comprend un appareil pour surveiller l'intégrité du carreau non cuit, qui comprend :
- un dispositif (155) pour appliquer, sur au moins une partie (P) du carreau non cuit (125), un liquide de contraste approprié pour modifier la couleur de ladite partie du carreau non cuit (125),
- un dispositif optique (160) approprié pour acquérir au moins une image de ladite partie (P) du carreau non cuit (125) après l'application du liquide de contraste et avant l'étape de cuisson, et
- une unité de traitement électronique (165) connectée audit dispositif optique (160) et configurée pour détecter si ladite image comprend au moins une ligne (Z) de couleur contrastée par rapport à la couleur adoptée par ladite partie (P) du carreau non cuit (125) à la suite de l'application du liquide de contraste.
